# EUROPEAN PATENT SPECIFICATION

(11) **EP 3 089 292 B1**
(45) Date of publication and mention of the grant of the patent: **27.05.2020**
(21) Application number: 14875175.3
(22) Date of filing: 18.11.2014
(51) Int. Cl.: H01T 19/04, H01T 23/00, A61L 9/22

(54) **DISCHARGE DEVICE AND AIR TREATMENT DEVICE**
ENTLADUNGSVORRICHTUNG UND LUFTAUFBEREITUNGSVORRICHTUNG
DISPOSITIF DE DÉCHARGE ET DISPOSITIF DE TRAITEMENT D'AIR

(30) Priority: 27.12.2013 JP 2013272461; 07.05.2014 JP 2014095764
(43) Date of publication of application: 02.11.2016
(73) Proprietor: Daikin Industries, Ltd., Osaka-shi, Osaka 530-8323 (JP)
(72) Inventor: SUZUMURA, Kei, Osaka 530-8323 (JP); TANAKA, Toshio, Osaka 530-8323 (JP); ENOKIDA, Tatsumi, Osaka 530-8323 (JP); YAMASHITA, Tetsuya, Osaka 530-8323 (JP); HARUNA, Shunji, Osaka 530-8323 (JP); SASAI, Yuta, Osaka 530-8323 (JP)
(74) Representative: Goddar, Heinz J.
(86) International application number: PCT/JP2014/005789
(87) International publication number: WO 2015/097978

(56) References cited:
- EP-A2- 1 279 439
- JP-A- 2005 135 894
- JP-A- 2005 296 916
- JP-A- 2011 104 558
- JP-A- 2011 104 558
- JP-A- 2013 045 531
- US-A1- 2009 193 976
- US-A1- 2012 048 792

## Description

### TECHNICAL FIELD

The present invention relates to a discharge device which produces a streamer discharge between a discharge electrode and a counter electrode, and relates to an air processing device.

### BACKGROUND ART

Discharge devices which provide a streamer discharge between a discharge electrode and a counter electrode have been known. Patent Document 1 discloses one of such discharge devices.

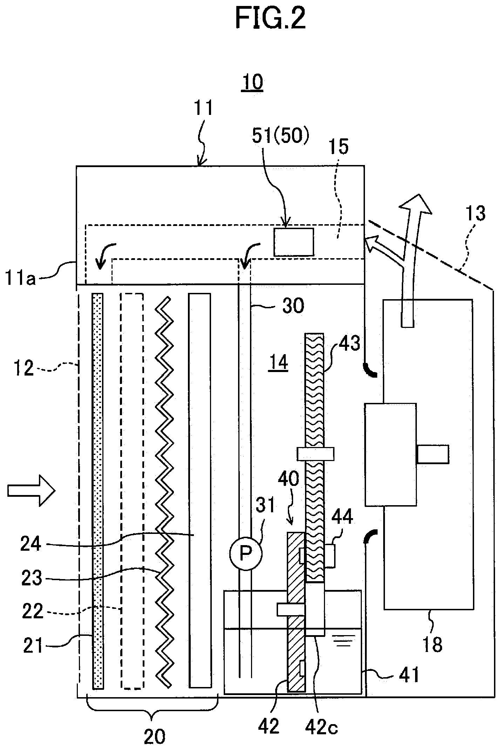

The discharge device of Patent Document 1 is mounted in an air cleaner. The discharge device has a discharge electrode and a counter electrode as illustrated in FIG. 2. The discharge electrode includes an electrode support member, a fixing member and a discharge needle. The tip of the discharge needle is opposed to the counter electrode.

In the discharge device, a streamer discharge is produced from the tip of the discharge needle of the discharge electrode toward the counter electrode when a voltage is applied from a power source to the discharge electrode and the counter electrode. Due to this streamer discharge, highly reactive substances (active species of, e.g., electrons, ions, radicals and ozone) are generated in the air. These active species decompose and remove harmful or odor substances in the air.

EP 1 279 439 A2 discloses a discharge device according to the preamble of claim 1.

US 2012/048792 A1 relates to a liquid treatment discharge unit, a humiditdy control device and a water heater. The humidity control device includes a discharge unit as a liquid treatment discharge unit for purifying water stored in a water tank. The discharge unit includes a discharge section for generating electric discharge to purify water and a power supply unit forming a power supply circuit of the discharge section. In the water tank the discharge section is arranged closer to a bottom surface of the water tank. The discharge section includes a pair of electrodes which are two electrodes. A pair of electrodes are a discharge electrode and a couneter electrode. The discharge electrode is provided so as to be immersed in water of the water tank. The discharge electrode is made of metal, and is formed in a rod-like shape or a linear shape extending toward the counter electrode. The discharge electrode is formed so as to have a circular cross section perpendicular to the axis, but the cross section perpendicular to the axis may have other shapes such as triangular shape, a rectangular shape and an oval shape.

### CITATION LIST

### PATENT DOCUMENT

Patent Document 1: Japanese Unexamined Patent Publication No. 2005-296916

### SUMMARY OF THE INVENTION

### TECHNICAL PROBLEM

In the discharge device disclosed in Patent Document 1, as illustrated in FIG. 2, a plurality of discharge needles are arranged on the same line. A streamer discharge produced from the tip of each discharge needle tends to spread outward with respect to a direction in which the discharge needle extends. Thus, in the known discharge device, a certain space needs to be provided between adjacent discharge needles to avoid interference of the streamer discharges spreading from the adjacent discharge needles. Thus, a large space is necessary to arrange the plurality of discharge needles, which may lead to an increase in size of the discharge device.

In view of the foregoing, it is therefore an object of the present invention to reduce the distance between adjacent discharge needles and thereby achieve a reduction in size of a discharge device.

### SOLUTION TO THE PROBLEM

A first aspect of the present disclosure is directed to a discharge device according to claim 1.

According to the first aspect of the present disclosure, the plurality of discharge needles (64) are arranged parallel to one another. If the discharge needles (64) are arranged parallel to one another, adjacent discharge needles are less likely to produce, from their tips, streamer discharges that interfere with each other, compared to the plurality of known discharge needles (64) arranged on the same line. This may achieve desired streamer discharges even if the tips of the adjacent discharge needles (64) are brought closer to each other.

According to the first aspect of the present disclosure, the plurality of rod-like discharge needles (64) are supported on the plate portion (67) extending in the arrangement direction of the discharge needles (64).A second aspect of the present disclosure is an embodiment of the first aspect of the present disclosure. In the second aspect, the plurality of discharge needles (64) are arranged parallel to the counter electrode (71).

According to the second aspect of the present disclosure, the discharge needles (64) are arranged substantially parallel to the counter electrode (71). Thus, even if the tip of any discharge needle (64) is melted and shortened due to the discharge, the distance between the tip of the discharge needle (64) and the counter electrode (71) does not change. This allows stable discharge between the discharge needles (64) and the counter electrode (71) for a long period of time.

A third aspect of the present disclosure is an embodiment of the second aspect of the present disclosure. In the third aspect, the plurality of discharge needles (64) each in a rod-like shape protrude laterally from both side edges of the plate portion (67).

A fourth aspect of the present disclosure is an embodiment of the third aspect of the present disclosure. In the fourth aspect, the discharge needle (64) which protrudes laterally from one side edge of the plate portion (67) and the discharge needle (64) which protrudes laterally from the other side edge of the plate portion (67) are arranged coaxially.

A fifth aspect of the present disclosure is directed to an air processing device including a discharge device (50) which purifies air, and the discharge device (50) is comprised of the discharge device of any one of the first to fourth aspects of the present disclosure.

### ADVANTAGES OF THE INVENTION

In a first aspect of the present disclosure, the plurality of discharge needles (64) are arranged parallel to one another, thereby making it possible to provide a stable streamer discharge even if adjacent discharge needles (64) are brought closer to each other. As a result, the discharge device may be reduced in size. In addition, the concentration of the active species and other substances generated by the discharge may be increased. This means that it is possible to provide a discharge device reduced in size and having high purification efficiencies.

In the second aspect of the present disclosure, the distance between the discharge needles (64) and the counter electrode (71) does not change even if the discharge needles (64) are melted and shortened due to long-term streamer discharges. This may maintain an optimal distance between electrodes, and allow stable streamer discharges for a long period of time.

In the third aspect of the present disclosure, the discharge needles (64) each formed in a rod-like shape protrude from a side edge of the plate portion (67). This enables a reduction in the thickness, weight and size of the discharge electrode (61).

### BRIEF DESCRIPTION OF THE DRAWINGS

[FIG. 1] FIG. 1 is a perspective view of the general configuration of an air processing device according to an embodiment, and shows a state in which a water tank is pulled out from a casing.
[FIG. 2] FIG. 2 is a vertical cross-sectional view generally illustrating the interior of an air processing device.
[FIG. 3] FIG. 3 is a block diagram illustrating an airflow in the air processing device.
[FIG. 4] FIG. 4 is a vertical cross-sectional view generally illustrating the interior of the air processing device closer to its front side.
[FIG. 5] FIG. 5 is a perspective view of a humidifier unit.
[FIG. 6] FIG. 6 is a perspective view illustrating a discharge electrode of the embodiment.
[FIG. 7] FIG. 7 is a front view of a discharge processing section of the embodiment.
[FIG. 8] FIG. 8 is a side view of the discharge processing section of the embodiment.
[FIG. 9] FIG. 9A is a general configuration of a fixed structure of the discharge electrode of the embodiment, for illustrating a state before attachment of discharge needles.
FIG. 9B is a general configuration of the fixed structure of the discharge electrode of the embodiment, for illustrating a state in which the discharge needles are attached. FIG. 9C is a general configuration of a fixed structure of the discharge electrode of the embodiment, for illustrating a state after completion of attachment of the discharge needles.
[FIG. 10] FIG. 10 is a general diagram for illustrating a fixed structure of a discharge electrode of a first variation.
[FIG. 11] FIG. 11 is a general diagram for illustrating a fixed structure of a discharge electrode of a second variation.
[FIG. 12] FIG. 12 is a front view of a discharge processing section according to a first example of another embodiment.
[FIG. 13] FIG. 13 is a side view of the discharge processing section according to the first example of another embodiment.
[FIG. 14] FIG. 14 is a perspective view of a discharge electrode according to a second example of another embodiment.
[FIG. 15] FIG. 15 is a perspective view of a discharge processing section in a purifying unit of a second embodiment.
[FIG. 16] FIG. 16 is a top view of the discharge processing section in the purifying unit of the second embodiment.
[FIG. 17] FIG. 17 is a cross-sectional view of the discharge processing section in the purifying unit of the second embodiment, taken along the A-A plane in FIG. 16.

### DESCRIPTION OF EMBODIMENTS

Embodiments of the present invention will be described in detail below, based on the drawings. The following embodiments are merely preferred examples in nature, and are not intended to limit the scope, applications, or use of the invention.

### «First Embodiment of Invention»

### <General Configuration of Air processing Device>

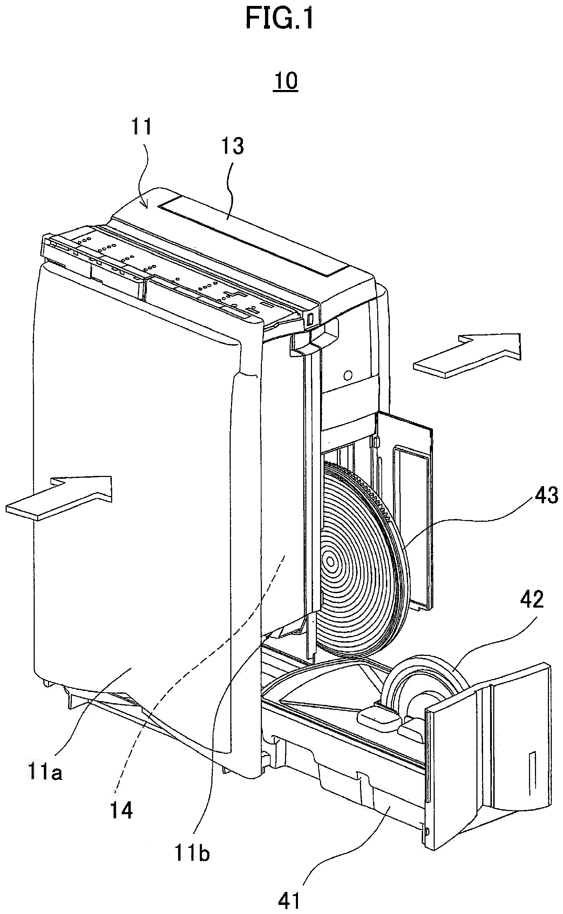

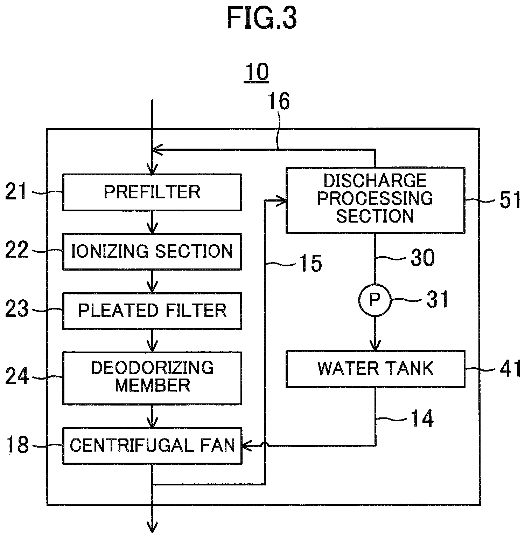

FIG. 1 is a perspective view of the general configuration of an air processing device according to a first embodiment of the present invention, and shows a state in which a water tank is pulled out from a casing. FIG. 2 is a vertical cross-sectional view generally illustrating the interior of the air processing device. FIG. 3 is a block diagram illustrating an airflow in the air processing device.

As illustrated in FIGS. 1-3, an air processing device (10) of the present embodiment includes various types of air purifying means (20) for purifying air, and a humidifier unit (40) which serves as a humidifying means for humidifying air.

The air processing device (10) includes a casing (11). The casing (11) is in a rectangular shape and is flat in the front-and-rear direction. The casing (11) is provided with a front panel (11a) on the front side thereof (on the left side of FIG. 1). Inlets (12) through which air is introduced into the casing (11) are formed in the front panel (11a) (see FIG. 2). The inlets (12) are formed, for example, in both lateral sides of the front panel (11a). The casing (11) is further provided, at a position closer to its upper rear side, with an outlet (13) for blowing out the air from inside the casing (11). An air path (14) is formed in the interior of the casing (11). Air flows through this air path (14) from the inlet (12) to the outlet (13).

As illustrated in FIGS. 2 and 3, the air path (14) is provided with a prefilter (21), an ionizing section (22), a pleated filter (23), a deodorizing member (24), the humidifier unit (40), and a centrifugal fan (18) sequentially from upstream to downstream of the air.

Further, an inflow end of a return path (15) is open at a position above the centrifugal fan (18) and below the outlet (13). That is, part of air flowing out of the air path (14) toward the outlet (13) flows into the return path (15). The return path (15) constitutes a space extending in the front-and-rear direction, and is divided from the air path (14). An outflow end of the return path (15) communicates with the upstream side of the prefilter (21). A discharge processing section (51) is provided in the return path (15).

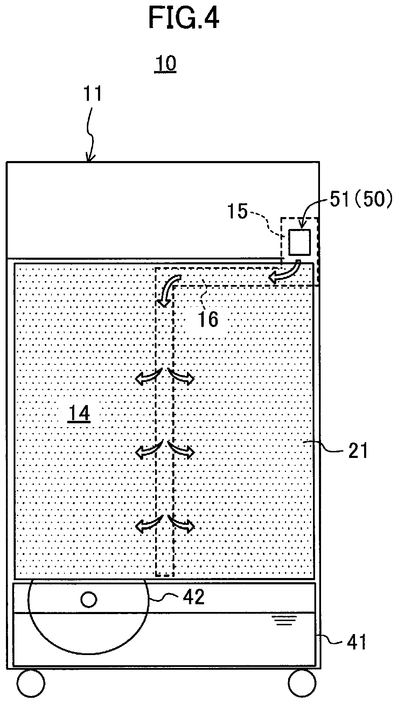

As illustrated in FIG. 4, the prefilter (21) is provided, on the front side thereof, with a guide path (16) that communicates with the return path (15). The guide path (16) is defined and formed, for example, by partition members provided on the back side of the front panel (11a). The guide path (16) is configured to guide the air which has flowed out of the return path (15) to a middle portion of the prefilter (21) in the width direction, and let the air flow to the lateral sides of the guide path (16) in order to send the air to the prefilter (21) (see arrows in FIG. 4). The return path (15) branches at downstream positions of the discharge processing section (51). One of the branched paths communicates with the guide path (16), and the other communicates with a transfer pipe (30) extending toward a water tank (41) of the humidifier unit (40). The transfer pipe (30) is provided, at a position along the passage, with an air-blow pump (31) for supplying air which contains active species generated in the discharge processing section (51) to the water in the water tank (41). Note that this air-blow pump (31) may be omitted.

### <Configuration of Air Purifying Means>

As illustrated in FIG. 2, the air processing device (10) includes the above-described prefilter (21), ionizing section (22), pleated filter (23) and deodorizing member (24) as the air purifying means (20) for purifying air.

The prefilter (21) constitutes a dust collection filter which physically catches relatively-large dust contained in the air.

The ionizing section (22) constitutes a dust charging means which charges dust in the air. The ionizing section (22) has, for example, a linear electrode and a plate-like electrode opposed to the linear electrode. The ionizing section (22) produces a corona discharge between the two electrodes when a voltage is applied to the electrodes from a power source. Due to this corona discharge, dust in the air is charged positively or negatively.

The pleated filter (23) constitutes a corrugated plate-like electrostatic filter. That is, the pleated filter (23) electrically attracts and catches the dust charged by the ionizing section (22). The pleated filter (23) may carry a deodorant material, such as a photocatalyst.

The deodorizing member (24) is formed of a honeycomb base material which carries, on a surface thereof, a deodorant for deodorizing air. Examples of the deodorant include an adsorbent which adsorbs a target component (e.g., odorous substances and harmful substances) in the air, and a catalyst for oxidizing and decomposing the target component.

### <Configuration of Humidifier Unit>

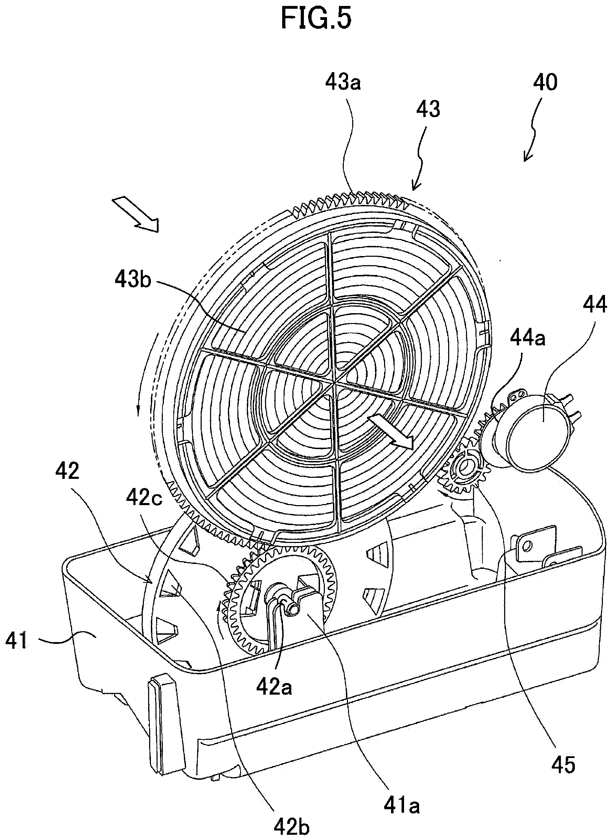

As illustrated in FIG. 5, the humidifier unit (40) includes a water tank (41) for storing water, a water wheel (42) for drawing up the water in the water tank (41), a humidifying rotor (43) which gives the water drawn up by the water wheel (42) to the air, and a drive motor (44) for rotatably driving the humidifying rotor (43).

The water tank (41) serves as a horizontally-oriented water container having an open upper end. The water tank (41) is installed in a lower space of the casing (11), and is freely loaded and unloaded through a draw-out opening (11b) of the casing (11) (see FIG. 1). This allows a user to refill the water tank (41) with humidifying water as necessary. Further, a bearing member (41a) for rotatably holding the water wheel (42) is vertically arranged on a bottom surface of the water tank (41).

The water wheel (42) is formed into an approximately disc-like shape that is flat in the front-and-rear direction, and a rotating shaft (42a) is provided so as to protrude from the center of the water wheel (42). The rotating shaft (42a) is rotatably supported on the upper end of the bearing member (41a). The water wheel (42) is rotatably arranged such that part (a predetermined portion including a lower end portion) of the water wheel (42) is dipped in the humidifying water in the water tank (41). The water wheel (42) serves as a rotating member. A plurality of rear-side recessed portions (42b) are formed in the water wheel (42) on its rear surface (i.e., the side surface facing the humidifying rotor (43)) around the rotating shaft. Each of the rear-side recessed portions (42b) serves as a recess for drawing up the humidifying water into the humidifying rotor (43) for humidification. Each of the plurality of rear-side recessed portions (42b) has an approximately trapezoidal opening whose width gradually increases toward the outside of the water wheel (42) in its radial direction. The width of the opening of each of the rear-side recessed portions (42b) in a circumferential direction is smaller than the width of the interior space of the rear-side recessed portion (42b) in the circumferential direction. Further, the inner wall of each of the rear-side recessed portions (42b) on its radially inner side gradually inclines such that the inner wall be closer to the center of the water wheel (42) toward the opening edge. The rear-side recessed portions (42b) are arranged at regular intervals in the circumferential direction at an outer peripheral portion of the water wheel (42) in the radial direction. During rotation of the water wheel (42), the positions of the rear-side recessed portions (42b) are shifted alternately between a position where the rear-side recessed portions (42b) are dipped in the water in the water tank (41) and a position where the rear-side recessed portions (42b) are taken out of the water. The rear surface of the water wheel (42) is also provided with an integrally-formed gear (42c) at a postion closer to the center of the water wheel (42). The gear (42c) is configured to engage with a driven gear (43a), described later, of the humidifying rotor (43).

The humidifying rotor (43) includes an annular driven gear (43a) and a disc-like moisture absorbing member (43b) fitted and held inside the driven gear (43a). The moisture absorbing member (43b) is made of unwoven fabric having water-absorbing properties. The humidifying rotor (43) is rotatably held, via a rotating shaft, at a position higher than the full water level of the water tank (41). In addition, the humidifying rotor (43) is arranged such that a predetermined portion including a lower end portion comes substantially in contact with the water wheel (42). In other words, the humidifying rotor (43) includes portions which overlap, in its axial direction, with the rear-side recessed portions (42b) of the water wheel (42). This structure allows the moisture absorbing member (43b) of the humidifying rotor (43) to absorb the humidifying water drawn up by the rear-side recessed portions (42b) of the water wheel (42).

The drive motor (44) has a drive gear (44a). The drive gear (44a) engages with the driven gear (43a) of the humidifying rotor (43) via a pinion (45). In other words, the drive gear (44a) rotated by the drive motor (44) causes rotation of the pinion (45) and the driven gear (43a), which further causes rotation of the water wheel (42) that engages with the driven gear (43a).

### <Configuration of Purifying Unit>

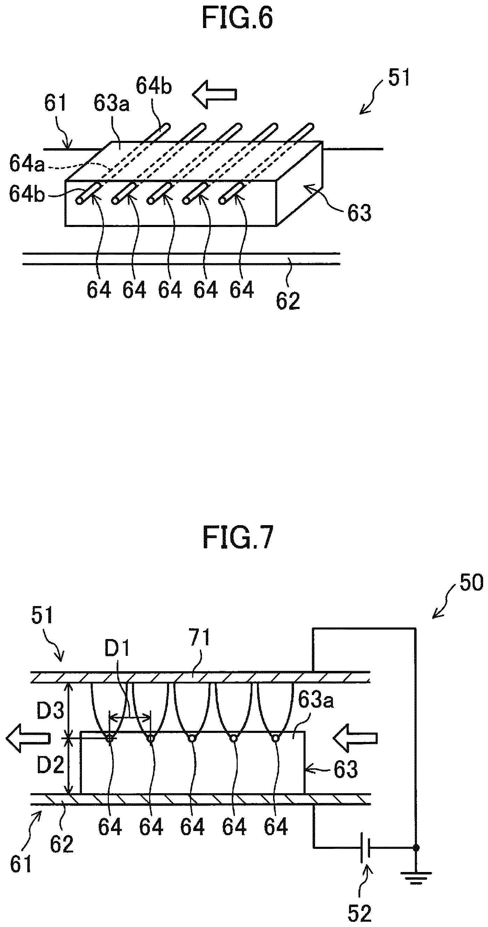

The air processing device (10) has a purifying unit (a discharge device (50)) for purifying air and the humidifying water. The purifying unit (50) includes the discharge processing section (51) having a discharge electrode (61) and a counter electrode (71), and a power source (52). The purifying unit (50) will be described in detail with reference to FIGS. 6-8.

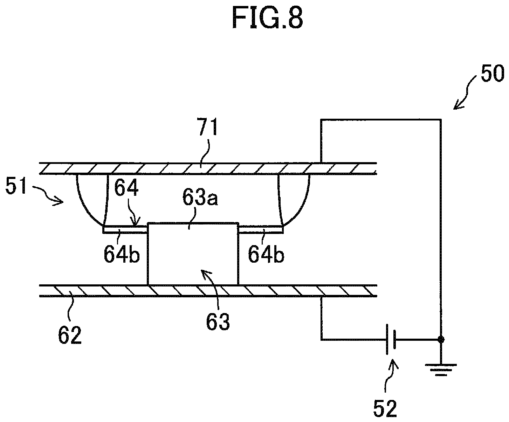

As illustrated in FIG. 8, the power source (52) is comprised of a high-voltage, direct-current power source, and applies a voltage between the discharge electrode (61) and the counter electrode (71). Specifically, the discharge electrode (61) is connected to a positive electrode side of the power source (52), and the counter electrode (71) is connected to a negative electrode side (more strictly, to the ground side) of the power source (52). The power source (52) conducts a so-called constant current control, in which a discharge current between the discharge electrode (61) and the counter electrode (71) is made constant.

The discharge electrode (61) includes an electrode support member (62), a base (63), and discharge needles (64). The electrode support member (62) has a plate-like shape, and supports the base (63) and the discharge needles (64). The electrode support member (62) of the present embodiment is formed of a metal material. Instead, the electrode support member (62) may be formed of a conductive resin material. The electrode support member (62) is arranged so as to be opposed to the counter electrode (71).

The base (63) is formed of a block-like member which projects from the electrode support member (62) to the counter electrode (71). Specifically, the base (63) has a parallelepiped shape that is flat in the direction in which the electrode support member (62) and the counter electrode (71) are opposed to each other (in the vertical direction of FIG. 6). The base end of the base (63) is fixed to the electrode support member (62). A plurality of discharge needles (64) are fixed to a projecting end (63a) of the base (63). The base (63) extends in a direction in which the plurality of discharge needles (64) are arranged. The electrode support member (62) of the present embodiment is formed of a metal material. Instead, the electrode support member (62) may be formed of a conductive resin material.

Each of the plurality of discharge needles (64) has a rod-like or linear shape, and is fixed to the projecting end (63a) of the base (63). The plurality of discharge needles (64) are arranged parallel to the electrode support member (62) and the counter electrode (71). The plurality of discharge needles (64) are arranged parallel to one another in a direction in which the base (63) extends. The lengths of the plurality of discharge needles (64) are the same. In the present embodiment, tips of the discharge needles (64) are arranged on the same line. Each of the plurality of discharge needles (64) has a fixed portion (64a) that is fixed to the projecting end (63a) of the base (63), and a pair of protruding portions (64b) that protrude toward both ends of the discharge needle (64) from the fixed portion (64a). The fixed portion (64a) of the discharge needle (64) is buried in the projecting end (63a) of the base (63). The counter electrode (71) has a plate-like shape, and is arranged so as to be parallel to the discharge needles (64). In the discharge processing section (51), air flows along an arrangement direction of the discharge needles (64). In other words, the plurality of discharge needles (64) are arranged in an air flow direction.

Distances between the elements of the discharge electrode (61) are set as follows. That is, the distance D1 between adjacent discharge needles (64) is 5.0 mm, and preferably 2.0 mm or more. Further, the distance D2 between each discharge needle (64) and the electrode support member (62) is 5.0 mm, and preferably 3.0 mm or more and 7.0 mm or less. The distance D3 between each discharge needle (64) and the counter electrode (71) is 5.0 mm, and preferably 3.0 mm or more and 7.0 mm or less. Setting these distances D1-D3 enables production of stable streamer discharges.

As a result of a potential difference applied to the discharge electrode (61) and the counter electrode (71) from the power source (52), a streamer discharge is produced from the tips of the protruding portions (64b) of the discharge needles (64) toward the counter electrode (71). Due to this streamer discharge, active species (such as radicals, ozone, high-energy electrons, and excited molecules) are generated in the air. Part of the air which contains the active species generated in the discharge processing section (51) is supplied to the prefilter (21) located at the most upstream of the air purifying means (20) through the guide path (16). The rest of the air which contains the active species is supplied into the water tank (41) through the transfer pipe (30). These active species react with target components in the air or water. These target components are thereby oxidized and decomposed, and are removed.

### -Operation-

The air processing device (10) of the present embodiment purifies air, using the above-described various air purifying means, and simultaneously humidifies room air, using the humidifier unit (40).

Specifically, first, the drive motor (44) rotatably drives the humidifying rotor (43) and the water wheel (42). When the centrifugal fan (18) is operated, the air in a room is introduced into the air path (14) through the inlet (12). A high voltage is applied to the electrodes (61, 71) of the discharge processing section (51) from the power source. Further, a voltage is applied to electrodes of the ionizing section (22) from the power source.

As illustrated in FIG. 2, the air which has flowed into the air path (14) passes through the prefilter (21), where dust is captured, and then passes through the ionizing section (22). In the ionizing section (22), a corona discharge is produced between the electrodes to charge the dust in the air. The air which has flowed out of the ionizing section (22) passes through the pleated filter (23). The pleated filter (23) electrically attracts and captures the charged dust. The air which has passed through the pleated filter (23) passes through the deodorizing member (24). In the deodorizing member (24), target components contained in the air are adsorbed by the adsorbent, or oxidized and decomposed by a catalyst.

Part of air on the blowout side (i.e., positive pressure side) of the centrifugal fan (18) flows into the return path (15) from the air path (14). The air flows through the return path (15) toward the front side, and passes through the discharge processing section (51). In the discharge processing section (51), a streamer discharge is produced between the electrodes (61, 71) opposed to each other. As a result, the above-described active species are generated due to the streamer discharge in the discharge processing section (51). Part of the air which contains the active species merges, through the return path (15), with the air flowing on the upstream side of the prefilter (21). As a result, the active species flows from the inflow end to the outflow end of the air path (14). This ensures time for the reaction of the active species with the target components in the air, and hence increases deodorizing properties.

On the other hand, the rest of the air flowing through the return path (15) while containing the active species is supplied to the water tank (41) through the transfer pipe (30) which branches from the return path (15). These active species oxidize and decompose the harmful substances contained in the water, and remove them from the water. The active species are also used for sterilizing the humidifying water.

During a humidifying operation, the air which has passed the deodorizing member (24) flows into the humidifying rotor (43). In the humidifier unit (40), the water wheel (42) rotates to supply the humidifying water in the water tank (41) to the moisture absorbing member (43b) of the humidifying rotor (43) as necessary. Specifically, the rear-side recessed portions (42b) of the water wheel (42) are dipped in the humidifying water stored in the water tank (41). This means that in the humidifying water, the humidifying water enters the rear-side recessed portions (42b) and is held therein. Each of the rear-side recessed portions (42b) holding the humidifying water is pulled up from the humidifying water, and is shifted further upward. As the rear-side recessed portion (42b) gradually comes close to the humidifying rotor (43), the humidifying water held in the rear-side recessed portion (42b) gradually flows out of the rear-side recessed portion (42b) due to its own weight. Almost all the humidifying water in the rear-side recessed portion (42b) will be flowing out by the time when the rear-side recessed portion (42b) reaches the uppermost position.

The humidifying water which has flowed out of the rear-side recessed portion (42b) comes in contact with the humidifying rotor (43) that is adjacent to this rear-side recessed portion (42b), and is absorbed by the moisture absorbing member (43b). In this manner, the humidifier unit (40) continuously supplies the humidifying water to the humidifying rotor (43).

Air flows through a water-filled portion of the humidifying rotor (43). Thus, the humidifying water contained in the moisture absorbing member (43b) is released in the air, thereby humidifying the air. The air purified and humidified in this manner is supplied into a room through the outlet (13).

### <Fixed Structure of Discharge Needle>

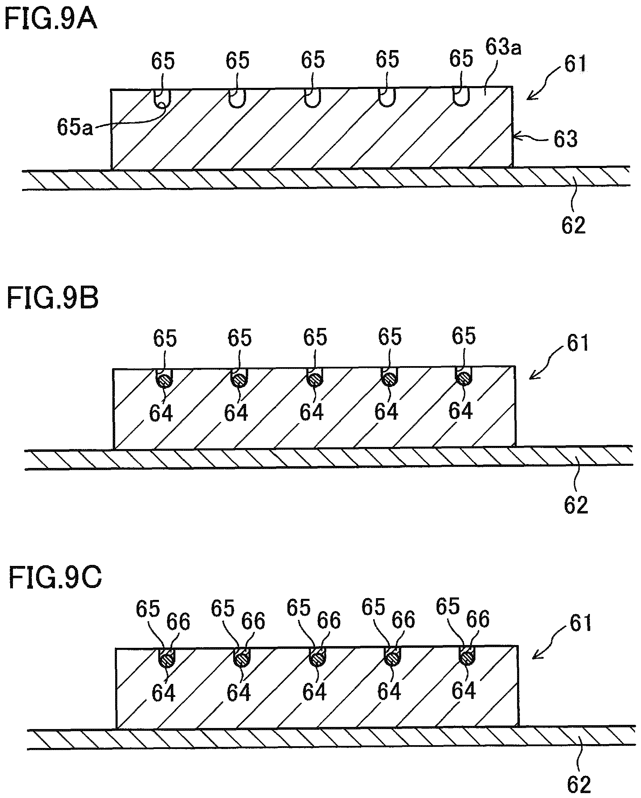

Now, how to fix the discharge needles (64) of the discharge electrode (61), and details of the fixed structure will be described with reference to FIGS. 9A to 9C. In the discharge electrode (61) of the present embodiment, the discharge needles (64) are fixed to the projecting end (63a) of the metal base (63) by soldering (brazing).

More specifically, as illustrated in FIG. 9A, a plurality of grooves (65) are formed in the projecting end (63a) of the base (63) of the discharge electrode (61) according to the present embodiment. These grooves (65) each have, on its bottom, an arc surface (65a) which allows the discharge needle (64) to be fitted therein. Each of the grooves (65) extends, on the projecting end (63a) of the base (63), from one to the other end in the width direction of the base (63).

Each of the discharge needles (64) is fitted in an associated one of the grooves (65), as illustrated in FIG. 9B. As a result, the discharge needles (64) are temporarily fixed to upper portions of the respective grooves (65).

Then, a brazing material (solder) is supplied on the discharge needles (64) which are fitted in the respective grooves (65), thereby fitting the discharge needles (64) to the inner surfaces of the respective grooves (65) via brazed portions (66). As a result, as illustrated in FIG. 9C, the discharge needles (64) are buried in the projecting end (63a) of the base (63).

### -Advantages of Embodiment-

In the above embodiment, the plurality of discharge needles (64) are arranged parallel to one another. If the discharge needles (64) are arranged parallel to one another, adjacent discharge needles (64) are less likely to produce, from their tips, streamer discharges that interfere with each other, compared to discharge needles arranged on the same line. This may achieve stable streamer discharges even with a reduced distance between the tips of the adjacent discharge needles (64). Thus, in the present embodiment, the discharge needles (64) may be brought closer to one another, compared to the known discharge needles. As a result, the discharge device may be reduced in size. In addition, the concentration of the active species and other substances generated by the discharge may be increased. This means that it is possible to provide a purifying unit (50) reduced in size and having high purification efficiencies.

In the above embodiment, the plurality of discharge needles (64) are fixed to the projecting end (63a) of the block-like base (63). In other words, the above embodiment adopts a structure that is different from a structure of the conventional cases in which a plate-like member is folded to fix discharge needles. This structure of the present embodiment may prevent application of force to the discharge needles (64) when the discharge needles (64) are attached to the base (63), and hence may prevent bending of the discharge needles (64). As a result, the distance between the tip of each discharge needle (64) and the counter electrode (71) may be set to a desired distance, and yields may be improved.

In the above embodiment, as illustrated in FIG. 9C, the discharge needles (64) are buried in the projecting end (63a) of the base (63). This structure ensures a satisfactory contact area between the base (63) and the discharge needles (64) more than in the conventional cases in which a plate-like member is folded to fix discharge needles. This may enable sufficient conductivity between the discharge needles (64) and the power source (52), and provide a stable discharge.

### -Variations of Embodiment-

In the above embodiment, the discharge needles (64) may also be fixed in a manner as described in the following variations.

### <First Variation>

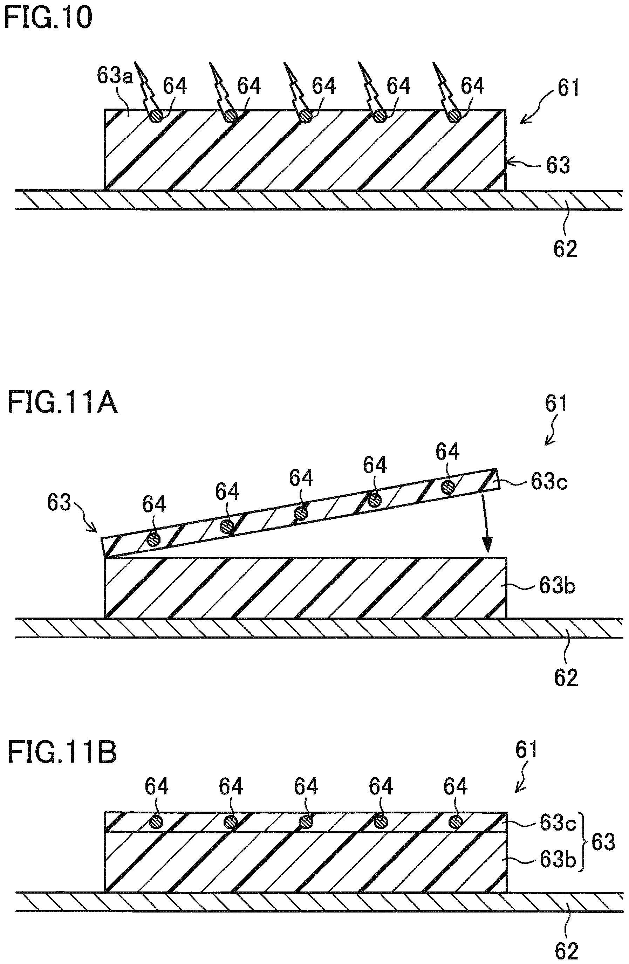

As illustrated in FIG. 10, the discharge needles (64) may be fixed to the projecting end (63a) of the base (63) by ultrasonic welding. Specifically, the base (63) of the first variation is made of a slightly-conductive resin material with a predetermined volume resistivity. Examples of the slightly-conductive resin material include polypropylene. It is recommended that the volume resistivity of the base (63) be 10⁻³ Ω·cm or more, and 10⁶ Ω· cm or less. In the present embodiment, the discharge needles (64) are welded to the projecting end (63a) of the base (63) by applying supersonic vibrations to the discharge needles (64) placed on the projecting end (63a) of the base (63).

In the first variation, too, the discharge needles (64) are fixed to the base (63) without applying force to the discharge needles (64). This may prevent bending of the discharge needles (64), and ensure an optimum distance between electrodes.

In the first variation, the slightly-conductive base (63) functions as a resistance of the positive electrode side of the power source (52). This may reduce a rise of the discharge current, and thus may prevent an abnormal discharge (i.e., a spark discharge) between the discharge electrode (61) and the counter electrode (71) in the purifying unit (50).

### <Second Variation>

As illustrated in FIG. 11, the discharge needles (64) may be insert-molded on the base (63). Specifically, the base (63) of the second variation has a base body (63b) and a surface layer portion (63c) which constitutes the projecting end. The base body (63b) and the surface layer portion (63c) are made of a slightly conductive resin material.

In the second variation, the slightly conductive resin and the discharge needles (64) are insert-molded as illustrated in FIG. 11A. As a result, the surface layer portion (63c) holding the discharge needles (64) therein is obtained. The discharge electrode (61) illustrated in FIG. 11B, in which the discharge needles (64) are buried in the projecting end (i.e., the surface layer portion (63c)) of the base (63), is formed by fixing the surface layer portion (63c) to the surface of the base body (63b).

In the second variation, too, the discharge needles (64) are fixed to the base (63) without applying force to the discharge needles (64). This may prevent bending of the discharge needles (64), and ensure an optimum distance between electrodes.

Moreover, the slightly conductive base (63) functions as a resistance of the positive electrode side of the power source (52) in the second variation, as well. This may reduce a rise of the discharge current, and thus may prevent an abnormal discharge (i.e., a spark discharge) between the discharge electrode (61) and the counter electrode (71) in the purifying unit (50).

### <Third Variation>

For example, the discharge needles (64) may be placed on the projecting end (63a) of the base (63), and thereafter the discharge needles (64) may be fixed to the base (63) with tape, an adhesive agent, etc.

### <<Other Embodiments>>

The above embodiment may have the following structures.

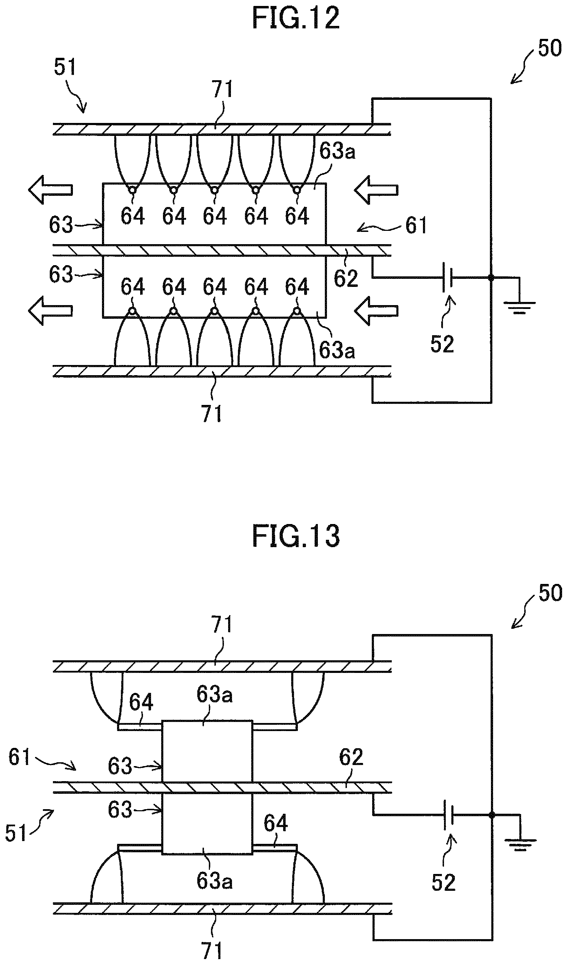

Another embodiment (a first example) illustrated in FIGS. 12 and 13 has a structure in which each of both sides of the electrode support member (62) in its thickness direction (both sides in the vertical direction of FIGS. 12 and 13) is provided with the base (63). In other words, two bases (63) share one electrode support member (62) in this example. This example requires a smaller number of components, compared to a case in which one electrode support member (62) is provided for each of the bases (63). Hence, the purifying unit (50) may be reduced in size.

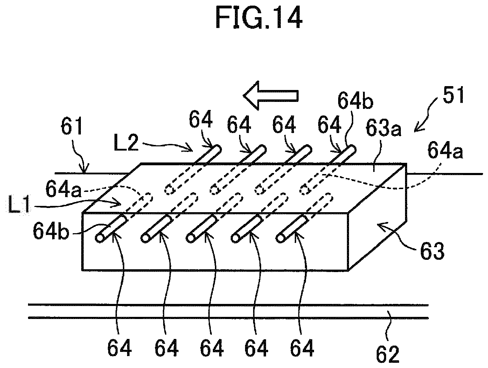

Another embodiment (a second example) illustrated in FIG. 14 has a different arrangement of the discharge needles (64) from the above embodiment. The discharge electrode (61) of this example is provided with discharge needles (64) of a first row (LI), which are arranged parallel to one another, and discharge needles (64) of a second row (L2), which are arranged parallel to one another. Each of the discharge needles (64) of the second row (L2) is located at an intermediate position between the discharge needles (64) of the first row (LI) arranged next to each other. Each of the discharge needles (64) of the first row (LI) has a fixed portion (64a) fixed to the base (63), and a protruding portion (64b) which protrudes from the fixed portion (64a) to the viewer's side of the FIG. 14. Each of the discharge needles (64) of the second row (L2) has a fixed portion (64a) fixed to the base (63), and a protruding portion (64b) which protrudes from the fixed portion (64a) to the side away from the viewer of FIG. 14.

The plurality of discharge needles (64) are arranged parallel to one another in this example as well. This structure allows the discharge needles (64) to be located closer to each other. As a result, in this example, too, the discharge device may be reduced in size, or air purification efficiency of the discharge device may be improved.

### <<Second Embodiment of Invention>>

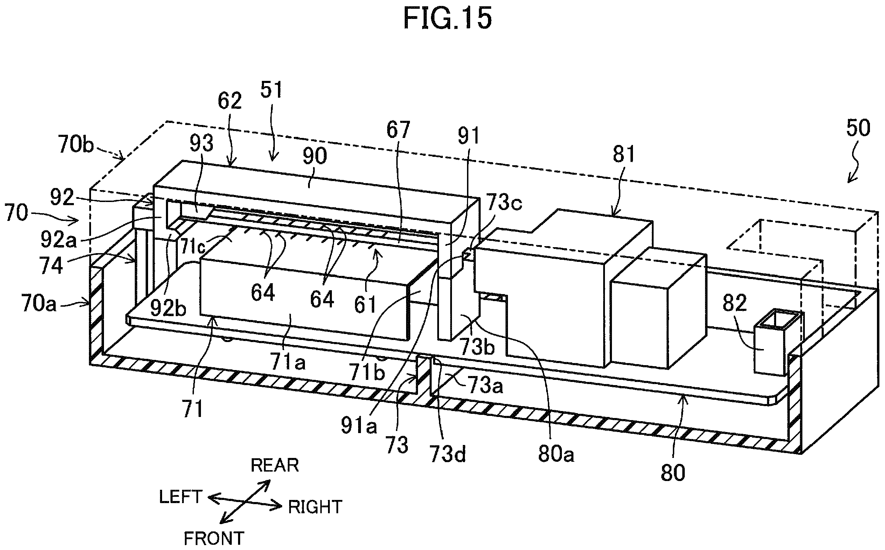

A purifying unit (i.e., a discharge device (50)) of the second embodiment of the present invention will be described with reference to FIGS. 15-17. The purifying unit (50) has a unit casing (70), a substrate (80), a power transformer (81), a connector (82) and a power source (not shown). In the following description, the viewer's side of FIG. 15 is referred to as a "front side," the side away from the viewer as a "rear side," the upper side as an "upper side," the lower side as a "lower side," the right side as a "right side," and the left side as a "left side."

The unit casing (70) is formed into a horizontally-oriented, parallelepiped box-like shape. The unit casing (70) includes a lower case (70a) arranged on the lower side and an upper case (70b) fitted to the upper side of the lower case (70a). The lower case (70a) is formed into a box-like shape with its upper end open. The lower case (70a) is made of an insulating material. The upper case (70b) is formed into a box-like shape with its lower end open. The upper case (70b) is made of an insulating material. The unit casing (70) is provided with a plurality of openings near the discharge processing section (51), for allowing air to flow in the front-and-rear direction. That is, in the unit casing (70), air passes through the discharge processing section (51) in the front-and-rear direction.

As illustrated in FIG. 15, an intermediate support (73) is provided at an intermediate position of the lower case (70a) in the right-and-left direction. The intermediate support (73) extends upward from the bottom surface of the lower case (70a). The intermediate support (73) includes a horizontally-oriented lower wall (73a) which protrudes upward from the bottom surface of the lower case (70a), an approximately square plate-like middle wall (73b) which extends upward from an intermediate portion, in the front-and-rear direction, of the lower wall (73a), and a protrusion (73c) which protrudes upward from an intermediate portion, in the front-and-rear direction, of the middle wall (73b). The lower wall (73a) is provided with substrate-receiving recesses (73d) which are recessed downward from upper edge portions of the lower wall (73a) at both lateral sides of the middle wall (73b).

The lower case (70a) is provided, at its left side end, with a lateral end support (74) which extends upward from the bottom surface of the lower case (70a). The lateral end support (74) is formed into a vertically-oriented rectangular column. The intermediate support (73) and the lateral end support (74) are made of an insulating material.

The substrate (80) is in a plate-like shape which extends across the interior of the lower case (70a) in the right-and-left direction. An insertion hole (80a) elongated in the front-and-rear direction is formed at a middle portion, in the right-and-left direction, of the substrate (80). The middle wall (73b) of the intermediate support (73) is inserted in the insertion hole (80a), and portions on the front and rear sides of the insertion hole (80a) are fitted in the substrate-receiving recesses (73d). The substrate (80) is held by the lower case (70a) in this manner. The power transformer (81) and the connector (82) are arranged on the substrate (80) at locations on the right side of the intermediate support (73). The connector (82) is connected to the power source via an electric wire not shown. The power transformer (81) boosts the voltage supplied to the connector (82). The boosted voltage is applied to a stabilizer (62) of the discharge electrode (61).

The purifying unit (50) includes a discharge processing section (51) which includes a discharge electrode (61) and a counter electrode (71). The counter electrode (71) is arranged on the substrate (80) at a location on the left side of the intermediate support (73). The counter electrode (71) is electrically connected to a grounding wire provided at the substrate (80), and is grounded. The counter electrode (71) is a member obtained by bending a flat plate into a square U shape (or U shape), and extends in the right-and-left direction. The counter electrode (71) includes side plates (71a, 71b) on the front and rear sides, and a top plate (71c) on the upper side. The counter electrode (71) is open downward. The top plate (71c) of the counter electrode (71) serves as a counter surface that is opposed to the discharge electrode (61). The counter electrode (71) of the present embodiment is made of a metal material. Instead, the counter electrode (71) may be made of a conductive resin material. The discharge electrode (61) is arranged above the counter electrode (71). The discharge electrode (61) includes a stabilizer (i.e., the electrode support member (62)), a plate portion (67), and discharge needles (64).

The stabilizer (62) is made of a conductive resin material. The stabilizer (62) is supported by the intermediate support (73) and the lateral end support (74) described above. The stabilizer (62) includes a beam (i.e., an intermediate portion (90)) extending along the plate portion (67), and first and second support portions (91, 92) provided at both ends of the beam (90) in the longitudinal direction,.

The beam (90) is arranged opposite to the counter electrode (71) with the plate portion (67) interposed therebetween. The beam (90) is arranged parallel to the plate portion (67) and is spaced from the plate portion (67). The first support portion (91) projects from the right end of the beam (90) toward the plate portion (67). The second support portion (92) projects from the left end of the beam (90) toward the plate portion (67).

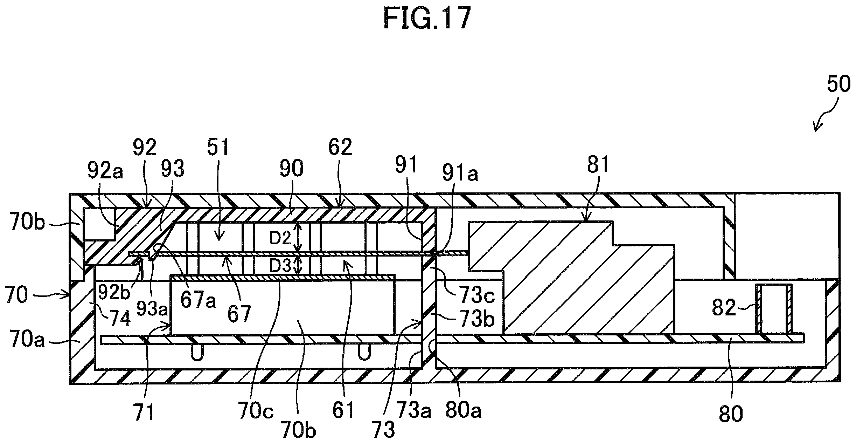

An intermediate recessed portion (91a) is formed at an intermediate portion, in the front-and-rear direction, of the projecting end of the first support portion (91). The protrusion (73c) of the intermediate support (73) is fitted in the intermediate recessed portion (91a). The right end of the plate portion (67) of the discharge electrode (61) is held by being sandwiched between the intermediate recessed portion (91a) and the protrusion (73c) (see FIG. 17).

The second support portion (92) includes a projecting wall (92a) which projects downward, and a hook (92b) bent inward (to the right side) from the projecting wall (92a). In addition, a sandwiching portion (93) is provided at an intermediate portion, in the front-and-rear direction, of the inner surface of the projecting wall (92a) so as to extend from the projecting wall (92a) to the beam (90). The sandwiching portion (93) is like a vertically-arranged plate which protrudes inward from the projecting wall (92a), and is slightly spaced from the hook (92b). The left end of the plate portion (67) of the discharge electrode (61) is held by being sandwiched between the hook (92b) and the sandwiching portion (93) (see FIG. 17).

Further, an engagement portion (93a) is provided at a lower right end of the sandwiching portion (93). The engagement portion (93a) protrudes downward. The plate portion (67) of the discharge electrode (61) is provided with an engagement hole (67a) at a position corresponding to the engagement portion (93a). Thus, in a state in which the plate portion (67) is held by the stabilizer (62), the engagement portion (93a) is fitted in the engagement hole (67a) of the plate portion (67). This determines a relative position between the stabilizer (62) and the plate portion (67), and further determines a relative position between the stabilizer (62) and the discharge needle (64).

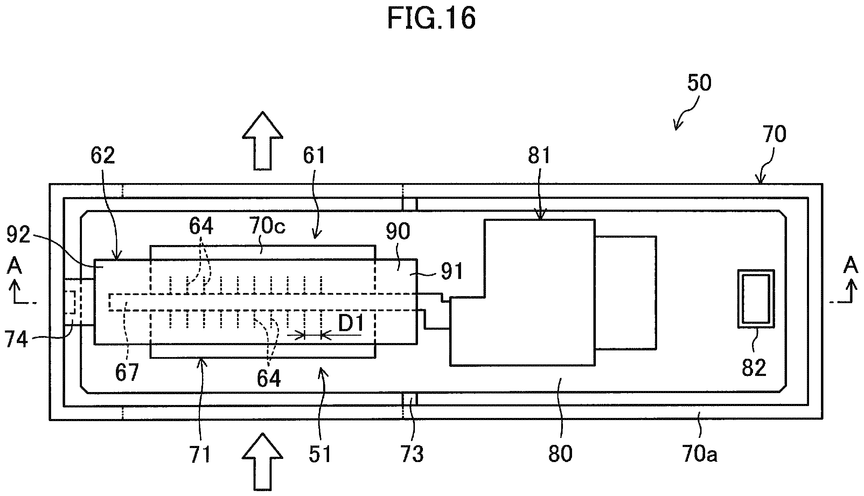

The plate portion (67) is formed into a narrow plate-like shape whose width (i.e., a length in the vertical direction of FIG. 16) is narrower than the widths of the stabilizer (62) and the top plate (71c) of the counter electrode (71). The plate portion (67) is arranged approximately parallel to the stabilizer (62) and the top plate (71c) of the counter electrode (71).

The discharge needles (64) each formed into a rod-like shape protrude laterally (in the front-and-rear direction) from side edges of the plate portion (67) in the front-and-rear direction. Thus, the discharge needles (64) extend in a direction of an airflow passing through the discharge processing section (51). The discharge needles (64) of the present embodiment are each formed into an elongated, square pole-like shape. In other words, each of the discharge needles (64) has an approximately square cross-section taken along a plane orthogonal to the axis of the discharge needle (64), and the cross-section is the same along the entire length of the discharge needle (64).

The discharge electrode (61) has ten discharge needles (64) at each of the side edges of the plate portion (67). The number of discharge needles (64) is merely an example and may be any number as long as it is a plural number. In the discharge electrode (61), the plurality of discharge needles (64) placed next to each other with the plate portion (67) interposed therebetween are arranged coaxially. The plurality of discharge needles (64) placed next to each other with the plate portion (67) interposed therebetween may also be arranged alternately. Similarly to the first embodiment, the discharge needles (64) on the respective side edges of the discharge electrode (61) are arranged parallel to one another. As illustrated in FIG. 16, the plurality of discharge needles (64) overlap with the beam (90) of the stabilizer (62) and the top plate (71c) of the counter electrode (71) in the vertical direction of the drawing.

The discharge needles (64) and the plate portion (67) are made of a metal material and integrally formed together. The discharge needles (64) and the plate portion (67) are formed on the same plane.

In the discharge processing section (51), each of the discharge needles (64) of the discharge electrode (61) and the top plate (71c) (i.e., the counter surface) of the counter electrode (71) are arranged approximately parallel to each other. The term "parallel" used herein means that the discharge needles (64) and the counter electrode (71) are substantially parallel to each other. Thus, if any of the discharge needles (64) warps due to dust or impurities adhering to the tip or other portions of the discharge needle (64), the discharge needle (64) and the counter electrode (71) may not be parallel to each other.

Distances between the elements of the discharge processing section (51) are set as follows. That is, the distance D1 (see FIG. 16) between adjacent discharge needles (64) is 5.0 mm, and preferably 2.0 mm or more. Further, the distance D2 (see FIG. 17) between each discharge needle (64) and the stabilizer (62) is 5.0 mm, and preferably 3.0 mm or more and 7.0 mm or less. The distance D3 (see FIG. 17) between each discharge needle (64) and the top plate (71c) of the counter electrode (71) is 5.0 mm, and 3.0 mm or more and 7.0 mm or less. Setting these distances D1-D3 enables production of stable streamer discharges.

### -Advantages of Second Embodiment-

The discharge electrode (61) of the present embodiment is configured such that the plurality of rod-like discharge needles (64) protrude from the side edges of the plate portion (67). This enables a reduction in the thickness, weight and size of the discharge electrode (61).

In the present embodiment, the stabilizer (62) is arranged opposite the counter electrode (71) with the plate portion (67) interposed therebetween. Since the beam (90) of the stabilizer (62) has the same polarity as that of the discharge needle (64), a streamer discharge does not spread toward the beam (90), but spreads toward the opposite side (i.e., toward the counter electrode (71)). This may achieve stable streamer discharges.

In addition, the plate portion (67) and the beam (90) are spaced from each other since no support members for supporting the discharge needles (64) are provided between the plate portion (67), from which the discharge needles (64) protrude, and the beam (90). This may prevent unstable discharges due to effects of electrical interference of a support member having the same polarity as the polarity of the discharge needles (64).

The other effects and advantages of the second embodiment are the same as, or similar to, those of the above-described first embodiment.

### INDUSTRIAL APPLICABILITY

As can be seen from the foregoing description, the present invention is useful as a discharge device which provides a streamer discharge between a discharge electrode and a counter electrode.

### DESCRIPTION OF REFERENCE CHARACTERS

- 50: Purifying Unit (Discharge Device)
- 52: Power Source
- 61: Discharge Electrode
- 62: Electrode Support Member (Stabilizer)
- 63: Base
- 63a: Projecting End
- 63c: Surface Layer Portion (Projecting End)
- 64: Discharge Needle
- 64a: Fixed Portion
- 64b: Protruding Portion
- 67: Plate Portion
- 71: Counter Electrode
- 90: Beam (Intermediate Portion)
- 91: First Support Portion (Support Portion)
- 92: Second Support Portion (Support Portion)

## Claims

1. A discharge device, comprising:
a power source (52);
a discharge electrode (61) having a plurality of discharge needles (64) each being in a linear shape or a rod-like shape; and
a counter electrode (71) formed along the plurality of discharge needles (64),
the discharge device providing a streamer discharge from a tip of each of the plurality of discharge needles (64) toward the counter electrode (71) as a result of receiving a potential difference applied to the discharge electrode (61) and the counter electrode (71) from the power source (52),
wherein the plurality of discharge needles (64) are arranged parallel to one another,
the discharge electrode (61) includes a plate portion (67) which extends in an arrangement direction of the plurality of discharge needles (64),
the plurality of discharge needles (64) each in a rod-like shape protrude laterally from a side edge of the plate portion (67), and
the discharge electrode includes an electrode support member (62), wherein the electrode support member (62) includes
an intermediate portion (90) which is arranged opposite the counter electrode (71) with the plate portion (67) interposed between the intermediate portion (90) and the counter electrode (71), **characterized in that** said intermediate portion (90) extends along the plate portion (67),
that it further comprises a pair of support portions (91, 92) which protrude from both ends of the intermediate portion (90) to the plate portion (67), and support the plate portion (67), and
that the intermediate portion (90) is arranged parallel to the plate portion (67) and is spaced from the plate portion (67).

2. The discharge device of claim 1, **characterized in that**
the plurality of discharge needles (64) are arranged parallel to the counter electrode (71).

3. The discharge device of claim 2, **characterized in that**
the plurality of discharge needles (64) each in a rod-like shape protrude laterally from both side edges of the plate portion (67).

4. The discharge device of claim 3, **characterized in that**
the discharge needle (64) which protrudes laterally from one side edge of the plate portion (67) and the discharge needle (64) which protrudes laterally from the other side edge of the plate portion (67) are arranged coaxially.

5. An air processing device, comprising:
a discharge device (50) which purifies air,
**characterized in that**
the discharge device (50) is comprised of the discharge device of any one of claims 1-4.

## Patentansprüche

1. Entladungsvorrichtung, umfassend:
eine Stromquelle (52);
eine Entladungselektrode (61) mit einer Vielzahl von Entladungsnadeln (64) jeweils in einer linearen oder stabförmigen Gestalt; und
eine Gegenelektrode (71), die entlang der Vielzahl von Entladungsnadeln (64) ausgebildet ist,
wobei die Entladungsvorrichtung eine Streamer-Entladung von einer Spitze von jeder der Vielzahl von Entladungsnadeln (64) zur Gegenelektrode (71) als Ergebnis des Empfanges einer Potenzialdifferenz bereitstellt, die von der Stromquelle (52) an die Entladungselektrode (61) und die Gegenelektrode (71) angelegt ist,
wobei die Vielzahl von Entladungsnadeln (64) parallel zu einander angeordnet ist,
die Entladungselektrode (61) einen Plattenabschnitt (67) enthält, der sich in einer Anordnungsrichtung der Vielzahl von Entladungsnadeln (64) erstreckt,
die Vielzahl von Entladungsnadeln (64) jeweils in einer stabförmigen Gestalt von einer Seitenkante des Plattenabschnitts (67) lateral vorsteht, und
die Entladungselektrode ein Elektrodenhalteelement (62) enthält, wobei das Elektrodenhalteelement (62) einen Zwischenabschnitt (90) enthält, der gegenüber der Gegenelektrode (71) angeordnet ist, wobei der Plattenabschnitt (67) zwischen dem Zwischenabschnitt (90) und der Gegenelektrode (71) eingefügt ist, **dadurch gekennzeichnet, dass** sich der Zwischenabschnitt (90) entlang des Plattenabschnitts (67) erstreckt,
dass sie ferner ein Paar Halteabschnitte (91, 92) aufweist, die von beiden Enden des Zwischenabschnitts (90) zum Plattenabschnitt (67) vorragen und den Plattenabschnitt (67) halten, und
dass der Zwischenabschnitt (90) parallel zum Plattenabschnitt (67) angeordnet und vom Plattenabschnitt (67) beabstandet ist.

2. Entladungsvorrichtung nach Anspruch 1, **dadurch gekennzeichnet, dass** die Vielzahl von Entladungsnadeln (64) parallel zur Gegenelektrode (71) angeordnet ist.

3. Entladungsvorrichtung nach Anspruch 2, **dadurch gekennzeichnet, dass** die Vielzahl von Entladungsnadeln (64) jeweils in einer stabförmigen Gestalt von beiden Seitenkanten des Plattenabschnitts (67) lateral vorragen.

4. Entladungsvorrichtung nach Anspruch 3, **dadurch gekennzeichnet, dass** die Entladungsnadel (64), die von einer Seitenkante des Plattenabschnitts (67) lateral vorragt, und die Entladungsnadel (64), die von der anderen Seitenkante des Plattenabschnitts (67) lateral vorragt, koaxial angeordnet sind.

5. Luftaufbereitungsvorrichtung, umfassend:
eine Entladungsvorrichtung (50), die Luft reinigt,
**dadurch gekennzeichnet, dass**
die Entladungsvorrichtung (50) aus der Entladungsvorrichtung gemäß einem der Ansprüche 1-4 besteht.

## Revendications

1. Dispositif de décharge, comprenant :
une source d'énergie (52) ;
une électrode de décharge (61) possédant une pluralité de pointes de décharge (64), chacune desquelles ayant une forme linéaire ou de tige ; et
une contre-électrode (71) formée le long de la pluralité de pointes de décharge (64),
le dispositif de décharge constituant une décharge continue d'un bout de chacune de la pluralité de pointes de décharge (64) vers la contre-électrode (71) du fait de la réception d'une différence de potentiel appliquée, par la source d'énergie (52), à l'électrode de décharge (61) et à la contre-électrode (71),
la pluralité de pointes de décharge (64) étant agencée de sorte que les pointes soient parallèles entre elles,
l'électrode de décharge (61) comprenant une partie de plaque (67) s'étendant dans une direction de l'agencement de la pluralité de pointes de décharge (64),
la pluralité de pointes de décharge (64), chacune des pointes, en forme de tige, faisant saillie latéralement d'un bord latéral de la partie de plaque (67), et
l'électrode de décharge comprenant un élément de support d'électrode (62), l'élément de support d'électrode (62) comprenant
une partie intermédiaire (90) agencée face à la contre-électrode (71), la partie de plaque (67) étant intercalée entre la partie intermédiaire (90) et la contre-électrode (71),
**caractérisé**
**en ce que** ladite partie intermédiaire (90) s'étend le long de la partie de plaque (67),
**en ce qu'**il comprend en outre une paire de parties de support (91, 92) faisant saillie des deux bouts de la partie intermédiaire (90) à la partie de plaque (67), et supportant la partie de plaque (67), et
**en ce que** la partie intermédiaire (90) est agencée parallèlement à la partie de plaque (67), et espacée de la partie de plaque (67).

2. Dispositif de décharge selon la revendication 1, **caractérisé en ce que** la pluralité de pointes de décharge (64) est agencée parallèlement à la contre-électrode (71).

3. Dispositif de décharge selon la revendication 2, **caractérisé en ce que** les pointes de la pluralité de pointes de décharge (64), chacune étant en forme de tige, font saillie latéralement des deux bords latéraux de la partie de plaque (67).

4. Dispositif de décharge selon la revendication 3, **caractérisé en ce que** la pointe de décharge (64), faisant saillie latéralement d'un bord latéral de la partie de plaque (67), et la pointe de décharge (64), faisant saillie latéralement de l'autre bord latéral de la partie de plaque (67), sont agencées de façon coaxiale.

5. Dispositif de traitement de l'air, comprenant :
un dispositif de décharge (50) purifiant l'air,
**caractérisé en ce que**
le dispositif de décharge (50) est composé du dispositif de décharge selon une quelconque des revendications 1 à 4.
